(19)

**Europäisches Patentamt**
**European Patent Office**
**Office européen des brevets**

(11)  **EP 3 467 489 B1**

(12)  **EUROPÄISCHE PATENTSCHRIFT**

(45) Veröffentlichungstag und Bekanntmachung des Hinweises auf die Patenterteilung:
**25.06.2025  Patentblatt 2025/26**

(21) Anmeldenummer: **17195267.4**

(22) Anmeldetag: **06.10.2017**

(51) Internationale Patentklassifikation (IPC):
**G01N 27/83** *(2006.01)*

(52) Gemeinsame Patentklassifikation (CPC):
**G01N 27/83**

(54) **VERFAHREN ZUR BESTIMMUNG DER GEOMETRIE EINER FEHLSTELLE UND ZUR BESTIMMUNG EINER BELASTBARKEITSGRENZE**

METHOD FOR DETERMINING THE GEOMETRY OF A DEFECT AND FOR DETERMINING A LOAD CAPACITY LIMIT

PROCÉDÉ DE DÉTERMINATION DE LA GÉOMÉTRIE D'UN DÉFAUT ET DE DÉTERMINATION DE LA CAPACITÉ DE CHARGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**10.04.2019  Patentblatt 2019/15**

(73) Patentinhaber: **Rosen IP AG**
**6370 Stans (CH)**

(72) Erfinder:
• **DANILOV, Andrey**
  **49809 Lingen (DE)**

• **PALMER, Johannes**
  **49809 Lingen (DE)**

(74) Vertreter: **Wischmeyer, André et al**
**Busse & Busse**
**Patent- und Rechtsanwälte**
**Partnerschaft**
**Großhandelsring 6**
**49084 Osnabrück (DE)**

(56) Entgegenhaltungen:
**US-A1- 2016 178 580   US-A1- 2016 187 523**
**US-A1- 2016 245 779   US-A1- 2017 176 629**
**US-B1- 6 316 937**

**Beschreibung**

Die vorliegende Erfindung betrifft ein Verfahren zur Bestimmung der Geometrie einer Fehlstelle sowie ein Verfahren zur Bestimmung einer Belastbarkeitsgrenze eines zumindest im Betrieb rückbelasteten Objekts.

Eine der wesentlichen Aufgaben von Pipelineinspektionen, insbesondere mit sogenannten intelligenten Molchen ist die Vorhersage sicherer Betriebsbedingungen, die sich aus dem Zustand der Pipeline ergeben. Insbesondere interessiert Pipeline-Betreiber der Zustand etwaiger Schweißnähte und die Anzahl und Größe von Fehlstellen. Fehlstellen sind beispielsweise Bereiche mit Metallverlusten aufgrund von Korrosion, Risse oder anderen Schwächungen einer Wand eines insbesondere zur Bevorratung oder zum Transport von flüssigen oder gasförmigen Medien vorgesehenen Objekts. Hierzu zählen beispielsweise Rohre, Pipelines oder Tanks.

Die Kenntnis des für eine Pipeline geltenden Maximaldrucks ("maximum burst pressure"), ab dem die Pipeline zerstört wird, ist relevant für die in der Pipeline einstellbaren Betriebsdrücke. Entsprechend ist die ist genaue Vorhersage dieses Drucks wichtig. Aktuell wird für die Berechnung dieses Grenzwerts eine Fehlstelle lediglich hinsichtlich ihrer Länge, Breite und Tiefe angenähert und mithin als Box betrachtet. Gerade für Metallverluste aufgrund von Korrosion auf der Außen- oder Innenseite einer Pipeline ist diese gebotene konservative Betrachtungsweise allerdings nachteilig, da die vereinfachten geometrischen Figuren die aktuelle Struktur der Fehlstelle notwendigerweise überschätzen. Dies führt zu der Unterschätzung des maximum burst pressures des Objekts und somit zur Unterschätzung der erlaubten Betriebsdrücke. Ein mit höherem Druck betreibbares Objekt wie eine Pipeline oder ein Gastank kann jedoch deutlich ökonomischer betrieben werden.

Es ist Stand der Technik für die Messung der Korrosion eines Objekts Scans magnetischer Streufluss-Daten (MFL-Daten) aufgrund von magnetischen Streuflussmessungen (MFL-Messungen) zur Bestimmung der Größe der (Korrosions-) Fehlstellen von speziell geschulten Personen auswerten zu lassen. Die in den Scans angezeigten Signale werden in Boxen parametrisiert und ausgewertet. Die für dieses auch Sizing genannte Bewerten der Messergebnisse notwendigen Annahmen sind einerseits proprietär. Andererseits ist die Interpretation der Messergebnisse stark durch die Erfahrungswerte der auswertenden Personen beeinflusst. Letztlich kann die Qualität der Vorhersagen lediglich anhand von Untersuchungen der Pipeline vor Ort sichergestellt werden. Dies geht wiederum mit hohen Kosten für die Betreiber einher. Der meistverbreitete Industriestandard API 1163 beschreibt die nachteiligen Effekte dieses vereinfachten Ansatzes. Es ist gut dokumentiert, dass die Qualität dieses Ansatzes stark von der Kenntnis der betrachtenden Personen abhängt. Die in der Praxis durchgeführten Ansätze sind immer eine durch subjektive Faktoren beeinflusste Interpretation der durch einen MFL-Messlauf erhaltenen Daten.

Darüber hinaus sind aus der wissenschaftlichen, d.h. theoretischen Betrachtung Ansätze bekannt, bei denen über sukzessive Variation und iterative Methoden einer angenommenen Fehlstellengeometrie eine möglichst genaue Simulation der gemessenen Signale über Vorwärtsmodelle erreicht werden soll. Hierbei kommen beispielsweise neuronale Netze zum Einsatz. Theoretisch können diese Ansätze Lösungen im Sinn einer sich ergebenden Fehlstellengeometrie ergeben, allerdings sind diese Lösungen nicht notwendigerweise realistisch. Dies gilt insbesondere für komplexe Datensätze, die unter bestimmten Inversproblemen zu unerwarteten, exotischen und falschen Lösungen führen. Während in genau definierten und abgegrenzten Testszenarien solche wissenschaftlichen Modelle eine Lösung des beschriebenen Problems in Form von Fehlstellengeometrien ergeben, ist dies für reale Messdaten, die eine Vielzahl von Störeinflüssen aufweisen, bislang noch nicht erfolgreich gewesen.

In der US 2016/0187523 A1 werden Eddy-Current-Sensoren beschrieben, deren Daten in einer nummerischen Inversion zur Bestimmung der Dicke von ineinander verschachtelten Pipeline-Rohren verwendet werden.

Die US 6,316,937 B1 offenbart die Verwendung unterschiedlicher Proportionalitätsgleichungen, in denen die auf Basis von MFL-Messungen gemessenen Spannungswerte gegenüber den jeweiligen Defekten materialabhängig aufgetragen werden.

Die US 2016/0178580 A1 offenbart die Verwendung einer vorab erstellten Gleichung zur Bestimmung der Dicke, Breite und Tiefe eines Defekts, der sich in MFL-Messungen wiederfindet.

Die US 2017/0176629 A1 offenbart eine weitere Methode zur Auswertung von Eddy-Current-Messungen in ineinander verschachtelten Bohrloch-Rohren.

Es ist daher Aufgabe der vorliegenden Erfindung, einen robusten Weg zur genauen Rekonstruktion einer realen Fehlstelle aus deren MFL-Messdatensatz aufzuzeigen und eine möglichst genaue Berechnung der Belastbarkeitsgrenze eines korrosionsbehafteten Objekts durchzuführen.

Die Aufgabe wird gelöst durch ein Verfahren gemäß Anspruch 1 sowie bezüglich der Bestimmung einer Belastbarkeitsgrenze durch ein Verfahren gemäß Anspruch 15. Vorteilhafte Ausgestaltungen der Erfindung sind den auf diese Ansprüche rückbezogenen Unteransprüchen sowie der nachfolgenden Beschreibung zu entnehmen.

Erfindungsgemäß ist vorgesehen, die Bestimmung der Geometrie einer oder mehrerer Fehlstellen mittels einer Mehrzahl von im Wettbewerb zueinander stehende Experten-Routinen mit jeweils zumindest einer eigenen Suchstrategie bzw. zumindest einem eigenen Algorithmus insbesondere parallel auf einer EDV-Einheit durchzuführen.

Ein solches erfindungsgemäßes Verfahren zur Bestimmung der Geometrie einer oder mehrerer realer, untersuchter Fehlstellen eines magnetisierbaren Objekts, insbesondere eines Rohres oder eines Tanks, mit einem auf Basis einer oder mehrere MFL-Messungen erzeugten Referenzdatensatzes des Objekts, umfasst eine zumindest teilweise Darstellung des Objekts mittels einer EDV-Einheit , insbesondere auf einem oder durch ein zumindest dreidimensionales Objektgitter, und umfasst weiter eine Bestimmung, insbesondere eine Erzeugung, einer anfänglichen Fehlstellengeometrie als Ausgangsfehlstellengeometrie, insbesondere auf dem Objektgitter oder auf einem zumindest zweidimensionalen Defektgitter,

eine Bestimmung, insbesondere eine Erzeugung, eines ersten MFL-Vorhersagedatensatzes als Ausgangsvorhersagedatensatz auf Basis der Ausgangsfehlstellengeometrie, insbesondere durch Simulation einer MFL-Messung oder Zuweisung eines MFL-Datensatzes,
und eine iterative Anpassung der Ausgangsfehlstellengeometrie an die Geometrie der realen Fehlstelle oder Fehlstellen mittels der EDV-Einheit, wobei diese Anpassung mittels mehrerer im Wettbewerb zueinander und vorzugsweise parallel zueinander laufender Experten-Routinen erfolgt, wobei in jeweiligen Experten-Routinen mittels wenigstens eines eigenen Algorithmus bzw. einer eigenen Suchstrategie und auf Basis der Ausgangsfehlstellengeometrie eine jeweilige Experten-Fehlstellengeometrie erzeugt wird.

Einen eigenen Algorithmus weist die Experten-Routine dann auf, wenn sich wenigstens einer der der Experten-Routine zur Verfügung stehenden Algorithmen zur Anpassung der Fehlstellengeometrie von den Algorithmen einer weiteren Experten-Routine zumindest teilweise unterscheidet. Vorzugsweise können stochastische Prozesse zur Differenzierung der Algorithmen unterschiedlicher Experten-Routinen verwendet werden. Jede Experten-Routine weist zumindest einen Algorithmus zur Anpassung der Fehlstellengeometrie auf, vorzugsweise stehen zumindest einer Experten-Routine mehrere Algorithmen zur Verfügung. Ebenfalls kann innerhalb einer Experten-Routine die Auswahl eines Algorithmus auf Basis stochastischer Prozesse erfolgen oder vorgegeben werden.

Erfindungsgemäß ist weiterhin vorgesehen, dass auf Basis der jeweiligen Experten-Fehlstellengeometrie ein jeweiliger Experten-Vorhersagedatensatz, insbesondere durch Simulation einer MFL-Messung oder Zuweisung eines MFL-Datensatzes, bestimmt wird, wobei die dem jeweiligen Experten-Vorhersagedatensatz zugrunde liegende Experten-Fehlstellengeometrie dann wenigstens mehreren und insbesondere allen der Experten-Routinen als neue Ausgangsfehlstellengeometrie zur weiteren Anpassung an die Geometrie der realen Fehlstelle bzw. der realen Fehlstellen zur Verfügung gestellt wird, wenn der Experten-Vorhersagedatensatz dem Referenzdatensatz ähnlicher ist als der Ausgangsvorhersagedatensatz. Anschließend, d.h. für die nächsten Vergleiche der jeweiligen Experten-Fehlstellengeometrien mit der neuen Ausgangsfehlstellengeometrie, wird der zur neuen Ausgangsfehlstellengeometrie gehörende Experten-Vorhersagedatensatz als neuer Ausgangsvorhersagedatensatz verwendet. Ein Maß für die Ähnlichkeit kann über eine Fitnessfunktion gebildet werden. Die iterative Anpassung mittels der Experten-Routinen erfolgt solange, bis ein Stopp-Kriterium erfüllt ist. Der Experten-Vorhersagedatensatz und der erste MFL-Vorhersagedatensatz zeigen beide zur jeweils angenommenen Fehlstellengeometrie gehörende MFL-Felder. Diese können berechnet oder simuliert sein.

Die Bestimmung des Experten-Vorhersagedatensatzes kann innerhalb des Arbeitsablaufs der Experten-Routine und/oder über ein von einer Überwachungsroutine separat angesteuertes Programmmodul erfolgen.

Die Bestimmung des Experten-Vorhersagedatensatzes auf Basis der jeweiligen Experten-Fehlstellengeometrie erfolgt weiterhin insbesondere dann, wenn noch keine ausreichend großen Datenbanken mit berechneten oder gemessenen MFL-Daten zu jeweiligen Fehlstellengeometrien zur Verfügung stehen, durch Simulation einer MFL-Messung, welche nachfolgend noch beschrieben wird. Alternativ kann der Experten-Fehlstellengeometrie auch ein MFL-Datensatz aus einer ausreichend umfangreichen Datenbank zur Verfügung gestellt werden. Auch ist eine kombinierte Herangehensweise, bei der zuerst in einer Datenbank nach bereits vorhandenen MFL-Daten gesucht wird und erst anschließend bei negativ verlaufener Suche eine Simulation durchgeführt wird, möglich. Dies kann insgesamt zu einer schnellen Bestimmung des jeweiligen Experten-Vorhersagedatensatzes führen.

Das erfindungsgemäße Verfahren wird vollständig und insbesondere automatisiert auf einer EDV-Einheit, die gegebenenfalls aus mehreren Rechnern bestehen kann, durchgeführt. Das zugehörige Rechenprogramm kann ein einziges Programm sein oder es kann sich um ein Programmpaket umfassend eine Mehrzahl von Programmmodulen handeln, die beispielsweise ressourcenbedingt verteilt auf unterschiedlichen EDV-Systemen oder -untereinheiten ablaufen und dort auf jeweiligen EDV-Medien gespeichert sein können. Ein Rechner weist insbesondere die typischen Mittel einer Datenverarbeitungseinheit wie ein oder mehrere Prozessoren, zumindest temporären Speicher (RAM), Datenkommunikationsmittel, Anzeigeund/oder Eingabeeinheiten auf. Während die Auswahl des Referenzdatensatzes vorzugsweise nutzergesteuert erfolgen kann, erfolgt die Bestimmung der Fehlstellengeometrie während der Iterationen automatisch. Vorzugsweise können vor der eigentlichen Iteration noch Programmparameter zur Auswahl der den Experten-Routinen zur Verfügung stehenden Algorithmen, der Bestimmung einer anfänglichen Fehlstellengeometrie, der Bestimmung des ersten Vorhersagedatensatzes und/oder eines Experten-Vorhersagedatensatzes, die jeweils MFL-Felder zeigen, festgelegt werden. Beispielsweise kann so festgelegt werden, ob die Bestimmung des Ausgangsvorhersagedatensatzes über eine Simulation einer MFL-Messung auf Basis eines das Objekt mit der Fehlstelle repräsentierenden Gitters erstellt oder über eine Regression aus einer Datenbank geladen werden soll. Insbesondere können im Fall der Simulation eines MFL-Feldes die zum Vergleich mit dem Referenzdatensatz notwendigen Parameter wie z.B. Richtung der Magnetisierung, Stärke der Magnetisierung, Abstand des Sensors von der Objektoberfläche und/oder Geschwindigkeit der Messvorrichtung festgelegt werden.

Durch die miteinander um Ressourcen der EDV-Einheit konkurrierenden Experten-Routinen, die mit ihren eigenen Suchstrategien jeweils eigene Lösungen zur Bestimmung der Geometrie der realen Fehlstelle suchen, wird insbesondere das bei den theoretischen Ansätzen aus dem Stand der Technik vorhandene Problem vermieden, dass isolierte Lösungen gefunden werden. Gegenüber der manuellen Auswertung der Datensätze ist die gefundene Lösung nicht nur deutlich besser sondern auch besser nachvollziehbarer und dokumentierbarer. Singuläre Lösungen, bei denen ein Algorithmus gleich welcher Art nicht weiterkommt und gleichwohl die Fehlstellengeometrie nicht realistisch wiedergegeben wird, werden auf diese Weise vermieden.

Für eine Simulation der zu einer Geometrie zugehörigen Streufluss-Daten wird in der Regel eine Darstellung des Objekts auf bzw. durch ein dreidimensionales Gitter notwendig sein. Auf diesem erfolgt die Darstellung erfolgt in dem Sinne zumindest teilweise, als dass zumindest der Teil des Objekts mit der Fehlstelle oder den Fehlstellen und vorzugsweise angrenzende Bereiche durch das oder auf dem Objektgitter repräsentiert sind. Alternativ kann die Bestimmung der Streufluss-Daten auch über eine Datenbankabfrage, beispielsweise mittels einer Regressionsfunktion erfolgen.

Obgleich das erfindungsgemäße Verfahren für die Bestimmung einer oder mehrerer Fehlstellen innerhalb eines Referenzdatensatzes gleichzeitig durchgeführt werden kann, wird nachfolgend der Einfachheit halber zumeist nur auf eine Fehlstelle Bezug genommen.

Die Fehlstellengeometrie kann für die MFL-Simulation bei einer geschickten Wahl der Darstellung des Objekts, insbesondere durch das oder auf dem zumindest dreidimensionalen Objektgitter, den Gitterelementen oder Gitterpunkten als Wert zugeordnet werden. Je nach Geometrie der jeweiligen Gitter können hierfür Interpolationen oder Gitteranpassungen notwendig sein.

Insbesondere werden die Fehlstellengeometrien, die die Basis für die Bestimmung der zugehörigen (MFL-, bzw. Experten-) Vorhersagedatensätze sind, allerdings durch Fehlstellentiefen, die beispielsweise die Tiefe einer Korrosion einer Objektoberfläche darstellen, auf den Gitterknoten eines zweidimensionalen Defektgitters definiert. Durch die zweidimensionale Darstellung der Fehlstellen können die Experten-Routinen deutlich schneller arbeiten als wenn die Anpassung der Fehlstellengeometrie auf einem dreidimensionalen Gitter durchgeführt wird.

Für die Simulation der MFL-Messung einer neuen Fehlstellengeometrie wird das insbesondere zweidimensionale Defektgitter vorzugsweise auf die Gitterpunkte des Objektgitters interpoliert, wobei die Oberfläche des darzustellenden Objekts an die Fehlstellentiefen der Fehlstellengeometrie angepasst wird. Die Simulation wird dann auf dem insbesondere dreidimensionalen Objektgitter berechnet. Alternativ kann die Streuflusssimulation ebenfalls auf einem zweidimensionalen Gitter oder mittels eines Regressionsmodells durchgeführt werden, welches auf eine Datenbank mit MFL-Datensätzen aufgrund von Finite-Elemente-Methode-Simulationen und/oder MFL-Messungen aufbaut.

Auf Basis der anfänglichen Fehlstellengeometrie, die beispielsweise über eine Look-up-Tabelle, einen Datenbankabgleich oder eine insbesondere einmaligen Ausführung einer Experten-Routine erhalten oder vorgegeben wird, wird ein erster MFL-Vorhersagedatensatz als Ausgangsvorhersagedatensatz bestimmt, insbesondere durch Simulation einer MFL-Messung. Die Simulation der MFL-Messung wird beispielsweise mittels eines Finite-Elemente-Modells als Vorwärtsrechnung durchgeführt. In der Simulation der Streuflussmessung werden die hierfür notwendigen Parameter entsprechend der realen Messung festgelegt. Dies betrifft insbesondere die Magnetisierungsrichtung, die Magnetfeldstärke und/oder den Abstand der Sensoren über der Oberfläche des Objekts. Auf Basis der anfänglichen Fehlstellengeometrie ergibt sich dann ein Ausgangsvorhersagedatensatz als simulierte Streuflussmessung. Dieser Datensatz könnte bereits mit dem Referenzdatensatz des Objekts verglichen werden, was allerdings zu Beginn der Iteration regelmäßig nicht zu aussagekräftigen Lösungen führt.

Eine separate Routine zur Festlegung einer anfänglichen Fehlstellengeometrie als Ausgangsfehlstellengeometrie ist nicht unbedingt notwendig, verringert allerdings die in den nachfolgenden Programmabläufen benötigte Rechenzeit. Alternativ kann die anfängliche Fehlstellengeometrie bereits das Ergebnis eines Durchlaufs einer Experten-Routine sein. Die anfängliche Fehlstellengeometrie kann weiterhin alternativ vorgegeben sein, beispielsweise durch eine komplett flache, sozusagen fehlstellenfreie Geometrie.

Die anfängliche Fehlstellengeometrie wird als Ausgangsfehlstellengeometrie in den iterativen Näherungsprozess der miteinander im Wettbewerb stehenden Experten-Routinen hineingenommen. Die Experten-Routinen selbst sind beispielsweise als eigene Programmmodule ohne direkte Interaktion miteinander voneinander unabhängig und können abhängig von einer Überwachungsroutine bzw. einem Hauptmodul mit Ressourcen, insbesondere mit Rechenzeit, ausgestattet werden.

Ausgehend von der in einem jeweiligen Experten-Modul entwickelten Experten-Fehlstellengeometrie wird für insbesondere jede dieser Geometrien wiederum Experten-Vorhersagedatensatz bestimmt, insbesondere durch Simulation einer eine MFL-Messung. Somit wird jeder Experten-Fehlstellengeometrie, die insbesondere als 2D-Datensatz von Tiefenwerten der Fehlstelle vorliegt, einen Experten-Vorhersagedatensatz als simulierte MFL-Messung erzeugt. Die Simulation der MFL-Felder auf Basis der jeweiligen Experten-Fehlstellengeometrien erfolgt entsprechend der vorbeschrieben Berechnung des Ausgangsvorhersage-Datensatzes. Insbesondere werden die Berechnungen auf Basis der jeweiligen Experten-Fehlstellengeometrien parallel durchgeführt. Dies kann mit der Aufbau einer Datenbank einhergehen, in der die zu jeweiligen Fehlstellen gehörenden Streuflussfelder abgespeichert sind mit dem Ziel, später und für andere ähnliche Daten Rechenzeit einsparen zu können.

Vor der Erzeugung des Experten-Vorhersagedatensatzes kann es vorteilhaft sein, für die Berechnung der Experten-Fehlstellengeometrie das zugrundeliegende Gitter, insbesondere das Fehlstellengitter, gegebenenfalls auch das Objektgitter anzupassen, insbesondere partiell zu verfeinern. Hierfür können insbesondere Mesh morphing-Techniken verwendet werden, bei denen das Objekt- oder Defektgitter durch Gitterpunktverschiebung und /oder -teilung insbesondere in Bereichen starker Gradienten verfeinert wird, um eine genauere Bestimmung der Geometrie oder nachfolgend eine genauere Simulation zu ermöglichen. In anderen Bereichen mit schwächeren Gradienten kann das Gitter gröber werden, um Rechenzeit zu sparen. So wird das verwendete Gitter automatisch für eine optimale Evaluierung der Fehlstellengeometrie angepasst. Gleichzeitig wird hierdurch eine signifikante Reduzierung der Anzahl der Unbekannten erreicht, so dass wiederum Rechenzeit gespart wird.

Wenn der Vergleich zwischen dem Referenzdatensatz und dem Experten-Vorhersagedatensatz einer Experten-Routine ergibt, dass dieser dichter an dem Referenzdatensatz liegt als der vorherige Ausgangsvorhersagedatensatz, so wird die zugehörige Experten-Fehlstellengeometrie als Ausgangsfehlstellengeometrie für die weiteren sowie für die entsprechende Experten-Routine zur Verfügung gestellt. Ausgehend von dieser Lösung können dann die weiteren Experten-Routinen in einem nächsten Iterationsschritt von dieser Geometrie starten, es sei denn, sie haben beispielsweise während der noch laufenden eigenen Fehlstellengeometriebestimmung eine wiederum bessere Lösung gefunden, die dann weiteren bzw. allen Experten-Routinen zur Verfügung gestellt wird.

Bei den im Wettbewerb zueinander stehenden Experten-Routinen werden vorzugsweise diejenigen hinsichtlich der zur Verfügung stehenden Ressourcen der EDV-Einheit bevorzugt, die wie nachfolgend beschrieben erfolgreicher in der Annäherung an die realen Messdaten sind als andere im Wettbewerb bestehende Experten-Routinen. Ressourcen der EDV-Einheit sind insbesondere die CPU- oder GPU-Zeit und/oder die oder eine Priorisierung in der Speicherbelegung.

Vorteilhafterweise laufen die Experten-Routinen (auf der EDV-Einheit) dergestalt im Wettbewerb zueinander, dass die Verteilung der Ressourcen der EDV-Einheit, insbesondere in Form von Rechenzeit, an eine jeweilige Experten-Routine in Abhängigkeit einer Erfolgsquote, bei der insbesondere die Anzahl der von der Experten-Routine berechneten und für eine oder mehrere andere Experten-Routinen zur Verfügung gestellten Ausgangsfehlstellengeometrien berücksichtigt wird, und/oder in Abhängigkeit einer Reduktion einer Fitnessfunktion, bei der insbesondere die Anzahl der für die Reduktion erzeugten Experten-Vorhersagedatensätze berücksichtigt wird, erfolgt. Der Wettstreit der Experten-Routinen ergibt sich insbesondere dadurch, dass von dem als Überwachungsroutine ausgebildeten Programmteil den jeweiligen Experten-Routinen dann vermehrt Ressourcen insbesondere in Form von Rechenzeit, vorzugsweise CPU- oder GPU-Zeit zugewiesen wird, wenn diese erfolgreicher sind als andere Experten-Routinen. Erfolgreich ist eine Experten-Routine dann, wenn sie eine mit einer zum Referenzdatensatz passenderen beispielsweise simulierten Streufeldmessung versehene Fehlstellengeometrie gefunden hat, die den anderen Experten-Routinen zur Verfügung gestellt wird.

Hieraus kann sich zum Beispiel ergeben, dass einzelne, besonders erfolgreiche Experten-Routinen mehr als 50% der gesamten zur Verfügung stehenden Rechenzeit erhalten, was die Gesamtdauer des erfindungsgemäßen Verfahrens deutlich reduziert. Gleichzeitig kann programmseitig vorgegeben werden, dass keine oder einzelne der Experten-Routinen nicht unter einen bestimmten Prozentsatz an Rechenzeit gelangen, um das Problem von singulären und exotischen Fehlstellengeometrien bzw. -ergebnissen aus den einzelnen Routinen zu vermeiden. So kann für den Fall, dass eine bis dahin erfolgreiche Experten-Routine nur eine lokale und keine globale Lösung findet, ein Ausweg aus der ansonsten im Stand der Technik vorkommenden Blockade-Situation gefunden werden.

Die Anpassung mittels der Experten-Routinen erfolgt solange, bis ein Stopp-Kriterium erfüllt ist. Hierbei handelt es sich beispielsweise um einen residualen Unterschied bezüglich der gemessenen und simulierten Messdaten. Es kann sich auch um ein externes Stopp-Kriterium beispielsweise auf Basis der zur Verfügung stehenden Rechenzeit oder um eine insbesondere vorgebbare Anzahl von Iterationen oder eine insbesondere vorgebbare oder vorgegebene oder aus der zur Verfügung stehenden Rechenzeit bestimmte Rechenzeit handeln.

Es hat sich herausgestellt, dass die Genauigkeit der Fehlstellenbestimmung durch das erfindungsgemäße Verfahren qualitativ verbessert ist. Eine sich hieraus ergebende Berechnung der maximalen Belastbarkeit zeigt, dass zum Beispiel Pipelines deutlich länger betrieben werden können. Die Genauigkeit der Fehlstellenbestimmung ist deutlich erhöht. Sich aus der simulierten Fehlstellengeometrie nach dem vor- und nachbeschriebenen Verfahren ergebene maximale Betriebsdrücke können bis zu 50% höher angesetzt werden, was für den Betrieb der Pipeline und deren Betreiber die Wartungs- und Unterhaltungskosten signifikant reduziert. Erstmalig lässt sich nunmehr auch für MFL-Datensätze eine adäquate Bestimmung des ASME B31G-2012 level 2 Ansatzes ("river bottom profile") für den "remaining

EP 3 467 489 B1

strength algorithm" realisieren.

Bevorzugt wird eine Verteilung der Ressourcen der EDV-Einheit insbesondere in Form von CPU-Zeit an eine jeweilige Experten-Routine in Abhängigkeit der Anzahl der von dieser Experten-Routine für alle Experten-Routinen zur Verfügung gestellten Ausgangsfehlstellengeometrien erfolgen. Hierbei kann es sich beispielsweise um eine Anzahl von Slots zur Berechnung der Experten-Vorhersagedatensätze in Form von simulierten MFL-Datensätzen handeln, um die Anzahl von parallel die Rechenaufgabe bearbeitenden Prozessorkernen oder dgl.. Es kann darüber hinaus im Rahmen des das erfindungsgemäße Verfahren realisierenden Computerprogrammprodukts vorgesehen sein, dass dieses sich auf die in den EDV-Einheiten vorhandenen Ressourcen in Form von Prozessorkernen, Speicherplatz, Speicherarchitektur, Grafikkarten etc. anpasst. Durch die Priorisierung besonders bevorzugter Experten-Routinen und deren Algorithmen wird die Erkennung der realen Fehlstellengeometrie deutlich schneller.

Um das Problem singulärer, lokaler Lösungen noch weiter zu minimieren, ist insbesondere vorgesehen, zur Bestimmung der Geometrie der Fehlstelle bzw. der Fehlstellen zusätzlich ein einen von dem ersten Referenzdatensatz linear unabhängigen, weiteren Referenzdatensatz zu verwenden. Die Datensätze sind dann linear unabhängig, wenn sie durch MFL-Messungen mit zueinander angewinkelten Magnetisierungen des Objekts erzeugt wurden. Die Magnetisierungen sind dann zueinander angewinkelt, wenn die jeweiligen mittleren induzierten magnetischen Feldstärken in dem untersuchten Bereich nicht parallel oder deckungsgleich verlaufen. Insbesondere liegt der Winkel zwischen 40° und 140°, vorzugsweise zwischen 80° und 100° sowie besonders bevorzugt bei 90°. Auf Basis der Ausgangsfehlstellengeometrie wird ein weiterer Ausgangsvorhersagedatensatz bestimmt, insbesondere mittels einer die lineare Unabhängigkeit, d.h. insbesondere die unterschiedlichen Magnetisierungen berücksichtigenden weiteren MFL-Simulation erzeugt, sowie eine Experten-Fehlstellengeometrie erst dann als Ausgangsfehlstellengeometrie verwendet, wenn die für beide unabhängigen Magnetisierungen bestimmten zugehörigen Experten-Vorhersagedatensätze den jeweiligen Referenzdatensätzen ähnlicher sind als die für die beiden Magnetisierungen bestimmten Ausgangsvorhersagedatensätze und/oder eine beide Experten-Vorhersagedatensätze berücksichtigende Fitnessfunktion verbessert ist. Durch die parallele bzw. miteinander einhergehende Verarbeitung der beiden linear unabhängigen Datensätze und der Verwendung einer Ausgangsfehlstellengeometrie, deren simulierte Messdaten im Hinblick auf eine Ähnlichkeit oder eine Fitnessfunktion insgesamt besser sein müssen, wird die Gefahr von Singularitäten weiter reduziert. Gleichzeitig verbessert sich die Qualität der für alle Experten-Routinen zur Verfügung stehenden Ausgangsvorhersagedatensätzen. Die Anzahl der Iterationen kann somit weiter reduziert werden.

Insbesondere werden der erste Referenz-Datensatz über eine MFL-Messung mit axialer Magnetisierung und der zweite Referenz-Datensatz über eine MFL-Messung mit Magnetisierungen in Umfangsrichtung des Rohres erzeugt. Hierbei sind die Magnetisierungen des Rohres oder auch eines Objekts rechtwinklig zueinander, so dass aus den Magnetstreuflussmessungen ein maximaler Informationsgehalt erhalten werden kann, der durch die gleichzeitige Betrachtung der entsprechenden Referenzdatensätze und der simulierten Experten-Vorhersagedatensätze während der Berechnung in vollem Umfang zur Verfügung steht. Die nachfolgend beschriebenen Verfahrensschritte verlaufen unter Berücksichtigung des Vorstehenden analog bei der Verwendung zweier aufgrund linear unabhängiger Magnetisierungen entstandener Referenzdatensätze.

Durch die Verwendung von Ausgangs- und/oder Experten-Vorhersagedatensätzen auf Basis eines Vorwärtsmodells zur Simulation der MFL-Messungen insbesondere mittels eines Finite-Elemente-Modells werden die MFL-Messungssimulationen schnell durchgeführt. Die Simulation der Streuflussmessungen auf Basis der Experten-Fehlstellengeometrien kann über ein eigenes Programmmodul realisiert werden, das insbesondere gesteuert und/oder überwacht von einer Überwachungsroutine, von den einzelnen Experten-Routinen separat aufgerufen wird. Es kann sich auch um mehrere Module handeln, die verteilt über einzelnen Rechnereinheiten einer jeweiligen Experten-Routine zur Verfügung gestellt werden.

Vorteilhafterweise wird die anfängliche Fehlstellengeometrie mittels einer Look-up-Tabelle, durch eine der Experten-Routinen und/oder durch einen maschinellen Lernalgorithmus erzeugt, was wie vorbeschrieben die Gesamtrechenzeit verbessert, insbesondere wenn zusätzlich bereits eine Gitteranpassung erfolgt.

Insbesondere kann die Verfeinerung des Objekt- und/oder des Defektgitters in den Bereichen stattfinden, in denen die Tiefe der simulierten Fehlstelle oder Fehlstellen einen Schwellwert überschreitet, wobei dieser Schwellwert vorgebbar sein kann, so dass nur Gradienten oberhalb einer bestimmten Größe zu einer Änderung des Gitters führen. Bei einer solchen Verfeinerung kann insbesondere die Gesamtzahl der Gradienten einer neuen Experten-Fehlstellengeometrie berücksichtigt werden, um einen Ausgleich zwischen der Adaption des jeweiligen Gitters, insbesondere des Objektgitters und den nachfolgenden Rechenoperationen zu erreichen.

Die Verfeinerung des Gitters mit dem Ziel einer Rechenzeitverringerung kann sowohl bereits auf Basis eines anfänglichen Referenzdatensatzes wie auch vor der Berechnung des Experten-Vorhersagedatensatzes erfolgen. Auch hierfür ist es möglich, ein separates Programmmodul oder einzelne Untermodule der jeweiligen Experten-Routinen vorzusehen.

Insbesondere reduziert die Verfeinerung des Objekt- und/oder Defektgitters besonders vorteilhaft durch Gitterpunktverschiebung und/oder -teilung die benötigte CPU-Zeit durch signifikante Reduktion der Anzahl der unabhängigen Variablen, die im Vorwärtsalgorithmus zur Simulation der MFL-Messung verwendet werden müssen.

Eine Gitterpunktverschiebung kann darüber hinaus für eine Anpassung von Objektoder Defektgitter verwendet werden.

Vorzugsweise wird als Maß für die Ähnlichkeit der Expertenvorhersage- und Referenzdatensätze eine Fitnessfunktion verwendet, um auf Basis von Standardroutinen und entsprechend schnell, d.h. bei Einsparung von Rechenzeit, einen Vergleich der simulierten und gemessenen Datensätze zu bewirken.

Insbesondere wird die Ausgangsfehlstellengeometrie in Form eines zweidimensionalen Datensatzes bzw. ein hierauf verweisender Zeiger in einem für alle Experten-Routinen zugänglichen Speicherbereich der EDV-Einheit abgelegt. Dieser Speicherbereich steht insbesondere wiederum unter Kontrolle einer Überwachungsroutine, so dass auch diesbezüglich eine Priorisierung einzelner Experten-Routinen vorgenommen werden kann.

Anstatt jedes Mal zu Beginn einer neuen Iteration auf die Ausgangsfehlstellengeometrie, die beispielsweise in einem zentralen und für alle Experten-Routinen zugänglichen Speicherbereich abgelegt ist, zu verwenden, kann zumindest eine Experten-Routine zu Beginn einer neuen Iteration unter Verzicht auf eine Übernahme der Ausgangsfehlstellengeometrie ihre eigene Experten-Fehlstellengeometrie anpassen. Hierfür kann eine Experten-Routine eine Funktionsvorschrift besitzen, in der beispielsweise in Abhängigkeit von in anderen Experten-Routinen verwendeten Suchstrategien gezielt eine gegenläufige Strategie ausgesucht wird. In einem solchen Fall können sich die Experten-Routinen indirekt beeinflussen. Ein solches Vorgehen kann insbesondere dann vorteilhaft sein, wenn festgestellt wird, dass eine bislang immer erfolgreiche Routine eine unrealistische Lösung bevorzugt. Diese kann beispielsweise anhand von unzulässigen Werten bezüglich der Tiefe einer Fehlstelle erkannt werden. Sofern eine Experten-Routine, die die Übernahme der Ausgangsfehlstellengeometrie nicht vornimmt, keine verbesserten Lösungen liefert, wird diese automatisch herunter priorisiert, so dass ihr zunehmend weniger Rechenzeit zur Verfügung gestellt wird.

Als Stopp- bzw. auch als Konvergenzkriterium kann vorzugsweise eine nach einer Mehrzahl von Iterationen ausbleibende und als substantiell zu bezeichnende Änderung der Ausgangsfehlstellengeometrie oder allgemeiner der Geometrie des Defektund/oder des Objektgitters angenommen werden. Die bis dahin gefundene Lösung ist dann die beste. Vorzugsweise wird das Stopp-Kriterium so gewählt, dass die beobachteten Variationen in der Streuflusssimulation, die zur Verfeinerung des Objektoder Defektgitters führen, substantiell, z.B. mit einem Faktor 2, unterhalb der Variationen liegen, die sich aus der individuellen Messstreuung ergeben und die beispielsweise auf der Basis sogenannter "Essential Variables" des API 1163 Standards individuell spezifiziert sind. Dadurch wird erreicht, dass die Genauigkeit des finalen Modells im Bereich der durch die Messung selbst vorgegebenen Genauigkeit liegt. Entsprechend wird vorzugsweise als Stopp-Kriterium ein Vergleich der Variation des Experten-Vorhersagedatensatzes mit der Messstreuung des realen Datensatzes verwendet. Das einen Programmstopp und insbesondere eine Ausgabe bzw. Speicherung der bis dahin berechneten Ausgangsfehlstellengeometrie bewirkende Stopp-Kriterium kann vorzugsweise durch vorab einstellbare Programmparameter spezifiziert werden.

Insbesondere stehen einer Experten-Routine mehrere Algorithmen zur Anpassung der Experten-Fehlstellengeometrie zur Verfügung. Hierbei kann es sich um Ansätze aus dem Bereich des maschinellen Lernens, der stochastischen Optimierung, empirischer und/oder numerischer Modellfunktionen handeln. Insbesondere können in den Experten-Routinen auch Erfahrungswerte auswertender Personen verwertet werden. Vorzugsweise werden in einer Experten-Routine die unterschiedlichen Algorithmen entweder zufallsgeneriert oder durch eine Auswahlfunktion ausgewählt. So wird ein ausreichend diverser Ansatz erzeugt, mit dem alle Lösungen zielgerichtet und unter Wettbewerbsbedingungen berücksichtigen werden können.

Die eingangs gestellte Aufgabe wird auch durch ein Verfahren zur Bestimmung einer Belastbarkeitsgrenze eines zumindest im Betrieb druckbelasteten und insbesondere als Öl-, Gas- oder Wasserpipeline ausgebildeten Objekts gelöst, wobei bei dem Verfahren ein eine oder mehrere Fehlstellen beschriebener Datensatz als Eingangsdatensatz in einer insbesondere als Vorwärtsmodellierung ausgebildeten Berechnung der Belastbarkeitsgrenze verwendet wird, wobei der Eingangsdatensatz zunächst gemäß einem vor- oder nachbeschriebenen Verfahren zur Bestimmung der Geometrie einer Fehlstelle erzeugt wird. Die vorteilhafte Darstellung der Fehlstellengeometrie, insbesondere als nicht-parametrisierte echte dreidimensionale Geometrie bzw. als zweidimensionale Fläche mit jeweiligen Tiefenwerten, macht bisher in der Industrie als notwendig vermutete Vereinfachungen überflüssig, so dass auch aus diesem Grund eine Steigerung der Genauigkeit der Fehlstellenbestimmung als Ganzes in bisher nicht erreichbarer Weise gewährleistet wird.

War bisher die Genauigkeit auf die Angabe des Punktes der Maximaltiefe der Fehlstelle beschränkt, wird nun das gesamte Profil mit hoher Genauigkeit ermittelt. Typischerweise wird die Genauigkeit der Maximaltiefe auf das in Abhängigkeit der Messgenauigkeit erreichbare Maß reduziert, also etwa $\pm$ 5% der Wandstärke im Vergleich zu bisher etwa $\pm$ 10% der Wandstärke bei dem Sizing nach dem eingangs beschriebenen Stand der Technik. Allerdings erreicht die Vorhersage der Belastbarkeitsgrenze in Abhängigkeit von der Geometrie der Fehlstelle gerade für kritische Fälle in der Genauigkeit Steigerungen von beispielsweise bisher $\pm$50% auf nun $\pm$5%. Der erfindungsgemäße Vorteil liegt somit insbesondere in einer erstmalig erreichten adäquaten Darstellung der Fehlstellengeometrie, die genau diese Steigerung erst ermöglicht.

Weitere Vorteile und Einzelheiten der Erfindung können der nachfolgenden Figurenbeschreibung entnommen werden. Schematisch dargestellt zeigt:

Fig. 1 eine Fehlstellenbestimmung nach dem Stand der Technik,

Fig. 2 eine schematische Darstellung eines erfindungsgemäßen Verfahrens,

Fig. 3 eine nähere Erläuterung eines Teils der Fig. 2,

Fig. 4 Vergleich eines Ergebnisses eines erfindungsgemäßen Verfahrens mit Messdaten,

Fig. 5 schematische Darstellung einer Gitterverfeinerung als Teil eines erfindungsgemäßen Verfahrens,

Fig. 6 Ergebnis eines erfindungsgemäßen Verfahrens.

Einzelne Merkmale der nachfolgend beschriebenen Ausführungsbeispiele können in Kombination mit den Merkmalen der unabhängigen Ansprüche auch zu erfindungsgemäßen Weiterbildungen führen.

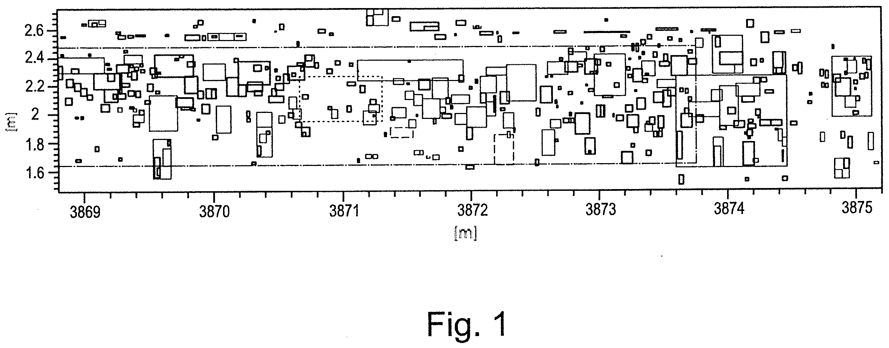

Im Stand der Technik wird die Auswertung von MFL-Daten eines Rohres gemäß Fig. 1 mittels der insbesondere auch erfahrungsbasierten Definition von Boxen vorgenommen. Die in der Abbildung dargestellten Boxen besitzen jeweilige Abmessungen in Länge, Breite und Tiefe. Die x- und y-Achse sind in Meter-Einheiten dargestellt ([m]). Eine Überprüfung der dieser Auswertung zugrunde liegenden tatsächlichen Fehlstellengeometrie mittels Laserscan, d.h. mittels einer direkten Messung, hat ergeben, dass der aufgrund der durch die MFL-Datenauswertung angenommenen Fehlstellengeometrie bestimmbare maximum burst pressure mit 4744,69 kPa nur 55.2% des aufgrund der tatsächlichen Geometrie berechneten maxium burst pressure beträgt. Durch den Stand der Technik liegt der Betriebsdruck für einen sicheren Betrieb der Pipeline, der sich aufgrund der erfahrungsbasierten Auswertung zu 3621,29 kPa ergibt, deutlich unterhalb eines möglichen sicheren Betriebsdrucks.

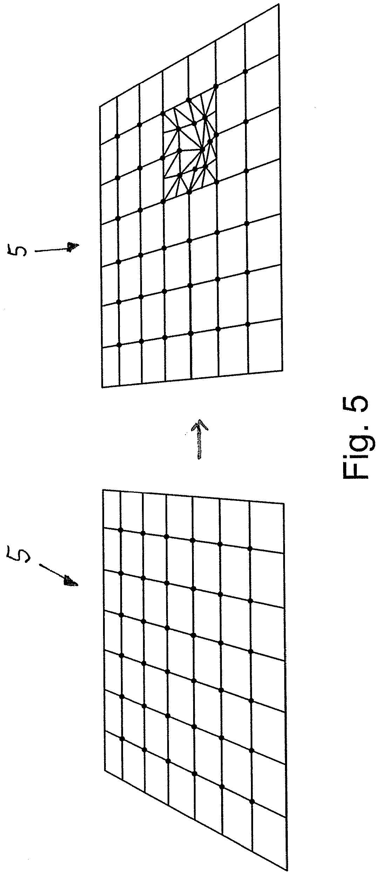

Beim erfindungsgemäßen Verfahren wird gemäß einem Ausführungsbeispiel die Oberfläche eines Rohres durch eine 2D Mesh-Oberfläche dargestellt. Die Fehlstellengeometrie kann als Vektor von Tiefenwerten D beschrieben werden, die auf einem Defektgitter 5 liegen (Fig. 5). Diese Fehlstellengeometrie wird auf Basis eines Ergebnisses einer die zu den jeweiligen Geometrien gehörenden MFL-Felder berücksichtigende Fitnessfunktion F(D) mit der Ausgangsfehlstellengeometrie verglichen. Hierbei wird angenommen, dass je geringer der Wert einer Fitnessfunktion ist, desto näher die angenommene Experten-Fehlstellengeometrie an der realen Geometrie ist:

$$F(D) = \sum_M \|H_{cal}(D) - H_m\| + R(D)$$

Hierbei ist M die Anzahl der gleichzeitig zu behandelnden Datensätze (reale MFL-Datensätze), $H_{cal}$ das Ergebnis einer Simulation der MFL-Messung, $H_m$ sind die gemessenen Daten aus der MFL-Messung (Referenzdatensatz), und R (D) ist ein Regularisierungsterm, der wie folgt angesetzt werden kann:

$$R(D) = \alpha |\nabla D|,$$

wobei $\alpha$ ein Skalierungsterm ist.

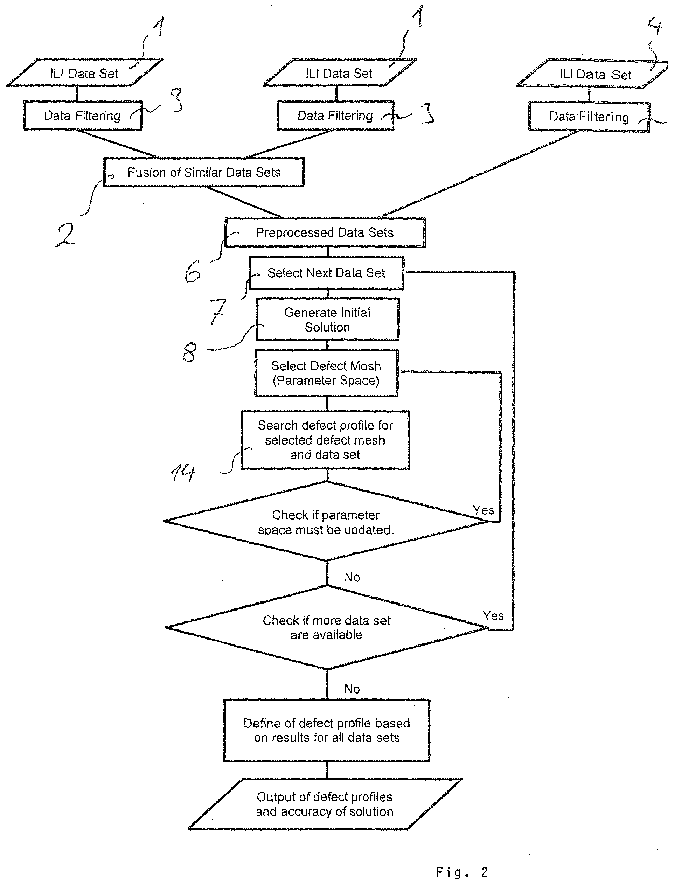

Der erfindungsgemäße Verfahrensablauf ist zumindest abschnittsweise nachfolgend gemäß Fig. 2 beschrieben, wobei eine Mehrzahl der parallel und im Wettbewerb stehenden Experten-Routinen 11 lediglich mit einem Block 14 beschrieben ist.

Als Eingangsdatensätze können beispielsweise mehrere Läufe desselben MFL-Pipelinemolches gemäß Box 2 zusammengeführt werden. Beide Datensätze 1 können zwecks besserer Zusammenführung vorher gefiltert werden und aneinander angeglichen werden (Verfahrensschritt 3), beispielsweise um etwaige Artefakte oder Hintergrundrauschen zu reduzieren. Darüber hinaus kann ein weiterer Datensatz 4, der auf Basis einer linear unabhängigen, weiteren Magnetisierung erzeugt wurde und ebenfalls zwecks Angleichung an identische Gitterstrukturen gefiltert wurde, verwendet werden, so dass gemäß Verfahrensabschnitt 6 zwei angeglichene Referenzdatensätze, die auf Basis von mit linearen unabhängigen Magnetisierungen versehenen Messläufen geschaffen wurden, vorhanden sind.

Genau aneinander angepasste Datensätze können gemeinsam behandelt werden, wobei das erfindungsgemäße Verfahren die gleichzeitige Behandlung der Datensätze durch Verwendung einer Fitnessfunktion, die die zusammengeführten Datensätze berücksichtigt, realisiert.

Im Schritt 7 wird ein erster der im Schritt 6 vorhandenen Referenzdatensätze zur Weiterbehandlung ausgewählt.

Hierzu wird in Schritt 8 zunächst eine anfängliche Fehlstellengeometrie als Ausgangsfehlstellengeometrie angenommen, insbesondere vorliegend generiert, die beispielsweise auf einem normalisierten Messsignal S(x,y)/(max S) basiert. Beispielsweise kann die Fehlstellengeometrie von einer Schwellwertfunktion abgeleitet werden, die die Amplitude an Gitterpunkten, bei denen das Signal größer als ein bestimmter Grenzwert / (z.B. 0,2) ist, berücksichtigt:

$$G(x,y) = \begin{cases} 0\,, if\ \dfrac{S(x,y)}{\max S} < l \\ \dfrac{S(x,y)}{\max S}\,, sonst. \end{cases}$$

Die vorstehende Näherung führt zu einer Anzahl von N-Fehlstellentiefenwerten an den jeweiligen Gitterpunkten:

$$D_i = i\ wt\ /\ N^*G,$$

mit *wt* als Dicke der Wand des Rohres. Für eine solche Fehlstellengeometrie wird die Fitnessfunktion berechnet und das Profil mit dem geringsten Funktionswert wird als Eingangslösung verwendet:

$$D_{init} = \arg\min F(D_i)$$

Diese Eingangslösung wird dann als Ausgangsfehlstellengeometrie für die einzelnen Expertenmodule zur Verfügung gestellt. Vorab kann mit dem Ziel einer Reduzierung der Rechenzeit die Anzahl der Parameterwerte (Elemente des Vektors D), die die Fehlstellengeometrie beschreiben, so klein wie möglich gehalten werden. Dies wird insbesondere über eine dynamische Gitteranpassung erreicht. Da die Anzahl der Tiefenwerte der Anzahl der Knotenpunkte im Defektgitter 5 entspricht, ist die Anzahl der Knoten gleichzeitig auch die Anzahl der Fehlstellenparameter. Beginnend bei einem vergleichsweise groben Gitter wird dieses sukzessive in relevanten Bereichen verfeinert.

Beispielhaft kann für einen vorgegebenen Knotenpunktabstand von beispielsweise 14 mm, einer hiermit einhergehenden Gitterzellengröße von 14 mm x 14 mm und Fehlstellengrenzwerten von 30%, 50% und 80% der Wanddicke die in Fig. 5 dargestellte Verfeinerung in dem relevanten Gitterbereich erreicht werden, wobei diejenigen Zellen sukzessive unterteilt werden, die die vorstehenden Tiefenwerte überschreiten. Die Gitterdeformation korreliert dann mit der angenommenen Fehlstellengeometrie, d.h. in Bereichen großer Gradienten befindet sich eine größere Anzahl an Gitterpunkten.

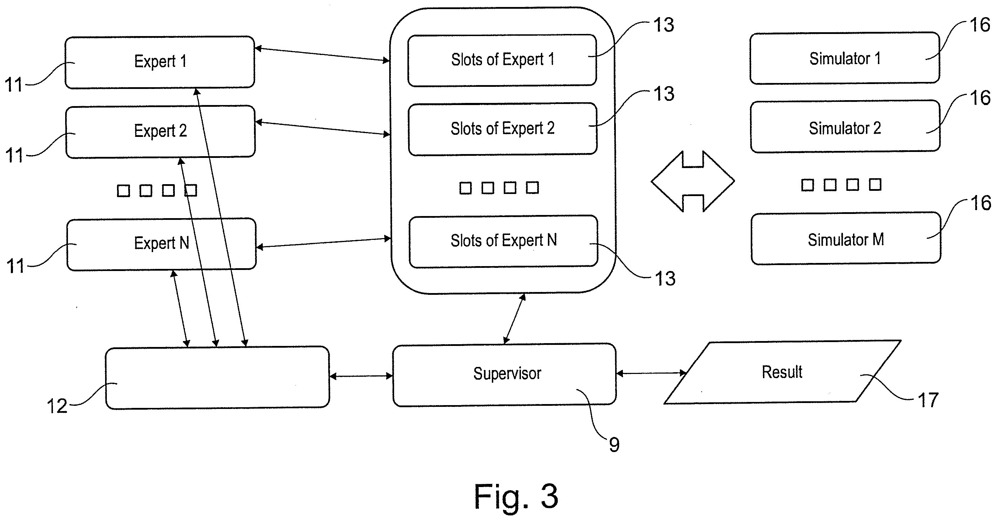

Auf der EDV-Einheit wird nach dem erfindungsgemäßen Verfahren der Ablauf des Arbeitsflusses einer Gruppe von Experten-Routinen 11, die miteinander im Wettstreit stehen, simuliert. Hierfür kann das Programm verschiedene Module aufweisen, die unabhängig voneinander und insbesondere nicht miteinander synchronisiert Daten in bestimmte Bereiche der EDV-Einheit einstellen können, damit diese dort weiter verarbeitet werden. Dies erfolgt insbesondere unter Aufsicht einer Überwachungsroutine 9 (Fig. 3a). Eine Mehrzahl von Experten-Routinen 11 hält somit in Abhängigkeit von dem vorstehend definierten Erfolg, d.h. z.B. der Anzahl der in einem gemeinsamen Speicherbereich 12 hineingeschriebenen Ausgangsfehlstellengeometrien eine Anzahl von Rechen-Slots 13, um jeweils Experten-Fehlstellengeometrien zu erzeugen und/oder zugehörige MFL-Simulationen durchführen zu können oder im Falle eines unabhängigen MFL-Simulationsmoduls durchführen zu lassen. Dies entspricht dem Block 14 nach Fig. 2, wobei dieser exemplarisch für mehrere Experten-Routinen 11 (Fig. 3a) steht. Ausgehend von den einzelnen Rechen-Slots 13 werden gemäß dem vorliegenden Ausführungsbeispiel die MFL-Simulationen der einzelnen Experten-Fehlstellengeometrien zwecks Erstellung der Experten-Vorhersagedatensätze ebenfalls unter Aufsicht der Überwachungsroutine 9 in den Simulationsmodulen 16 durchgeführt. Je mehr Slots 13 für eine Experten-Routine zur Verfügung steht, desto größer ist der Anteil an EDV-Ressourcen für diese Experten-Routine. Vorzugsweise ist die Anzahl der zur Durchführung von MFL-Simulationen Programmmodulen gleich der Anzahl der Slots. Die Überwachungsroutine 9 überwacht die Anzahl der Iterationen und die sich hieraus ergebenden Änderungen der Ausgangsfehlstellengeometrie und überwacht weiter, ob ein zugehöriges Stopp-Kriterium erreicht ist. Anschließend wird das Ergebnis gemäß Block 17 ausgegeben.

Die Anzahl der für eine Experten-Routine 11 zur Verfügung stehenden Rechen-Slots 13 und die anschließend zur Verfügung gestellten Simulationsroutinen können dergestalt variieren, dass eine erste Experten-Routine beispielhaft bis zu 50% der für die Rechen-Slots und Simulationsroutinen zur Verfügung stehenden gesamten Rechenzeit ausnutzen kann.

Im Speicherbereich 12 werden wie dargestellt die Ausgangsfehlstellengeometrien gespeichert. Hierbei kann es sich um einen den Experten-Routinen 11 zugänglichen Speicherbereich handeln. Dort können ebenfalls Log-Dateien der Experten- Routinen 11 und Überwachungs-Routine 9 sowie Anweisungen an die Experten-Routinen 11 hinterlegt werden, die von diesen dann selbständig umgesetzt werden. Beispielsweise kann es sich hierbei um einen Interrupt-Befehl handeln, der bei Erreichen des Stopp-Kriteriums gesetzt wird.

Vorzugsweise sind die Experten-Routinen 11 unabhängige Programmmodule, die neue Experten-Fehlstellengeometrien erzeugen und in die Simulationsroutinen 16 einstellen. Weiterhin kann in den Experten-Routinen 11 die eingangs dargestellte Fitnessfunktion auf Basis des Experten-Vorhersagedatensatzes erzeugt und mit dem im Bereich 12 abgelegten Ausgangsvorhersagedatensatz verglichen werden. Sofern der Experten-Vorhersagedatensatz dem Referenzdatensatz bzw. bei linear unabhängigen Messdatensätzen dann entsprechend den beiden Referenzdatensätzen insgesamt ähnlicher ist als der im Bereich 12 abgelegte Datensatz, wird dieser Experten-Vorhersagedatensatz dann als neuer Ausgangs-Vorhersagedatensatz verwendet.

Beispielsweise wird in den Experten-Routinen 11 eine neue Fehlstellengeometrie zufallsbasiert erzeugt. Hierfür können Maschinenlernalgorithmen oder empirische Regeln verwendet werden. Vorteilhafterweise ist jedoch zur weiterhin verbesserten Konvergenz der Lösungen die Realisierung zumindest zweier Basis-Experten-Routinen wie nachfolgend beschrieben vorgesehen.

Diese vorzugsweise immer bei einem erfindungsgemäßen Verfahren realisierten Suchstrategien basieren auf einer angenommenen Wahrscheinlichkeitsverteilung p(x,y) von Gitterpunkten, deren Tiefenwert eine maximale Reduktion der Fitnessfunktion ergeben. Die Wahrscheinlichkeitsfunktion wird verwendet um N Gitterpunkte $(x_n,y_n)$ zu identifizieren. An jedem der betrachteten Punkte wird die Tiefenfunktion, die beispielsweise die Tiefe der Korrosion an der Gitterstelle beschreibt, um $\Delta D$ geändert, wobei das Vorzeichen der Änderung zufallsgeneriert verteilt wird. Auch die Anzahl der ausgewählten Punkte N kann zufallsbasiert gewählt werden:

$$D_{new}(x,y) = \begin{cases} D(x_n, y_n) \mp \Delta D, \text{für ausgewählte Punkte} \\ D(x,y), \text{sonst} \end{cases}$$

Mit einer Auswahl der Wahrscheinlichkeitsfunktion p (x,y) können unterschiedliche Expertenstrategien realisiert werden, beispielhaft:

$$p(x,y) = \frac{D(x,y)}{\|D(x,y)\|}$$

Dieser Algorithmus realisiert eine Variation der Fehlstellentiefen, bei dem die Gitterpunkte mit der größten Tiefe bevorzugt werden. Eine andere Strategie kann wie folgt aussehen:

$$p(x,y) = \frac{H_{the\ best}(x,y) - H_m(x,y)}{\|H_{the\ best}(x,y) - H_m(x,y)\|}$$

Ein solcher Algorithmus variiert die Fehlstellengeometrie an Positionen, bei denen das simulierte Messsignal für die beste bekannte Lösung den größten Unterschied zum gemessenen Signal besitzt.

Basierend hierauf können durch Variationen der Anzahl der zu betrachtenden Gitterpunkte und des $\Delta D$ unterschiedliche Experten-Routinen bzw. deren Algorithmen aufgebaut werden. Beispielhaft können die nachfolgenden sechs Experten-Routinen verwendet werden:

1.

$$p(x,y) = \frac{D(x,y)}{\|D(x,y)\|}, \text{N = 1 and } \Delta D = 1\% \text{ Wanddicke}$$

2.

$$p(x,y) = \frac{D(x,y)}{\|D(x,y)\|}, \text{N = 2 and } \Delta D = 5\% \text{ Wanddicke}$$

3.

$$p(x,y) = \frac{D(x,y)}{\|D(x,y)\|}, \text{N = 3 and } \Delta D = 5\% \text{ Wanddicke}$$

4.

$$p(x,y) = \frac{H_{the\ best}(x,y) - H_m(x,y)}{\|H_{the\ best}(x,y) - H_m(x,y)\|}, \ \text{N} = 1 \text{ and } \Delta D = 1\% \text{ Wanddicke}$$

5.

$$p(x,y) = \frac{H_{the\ best}(x,y) - H_m(x,y)}{\|H_{the\ best}(x,y) - H_m(x,y)\|}, \ \text{N} = 2 \text{ and } \Delta D = 5\% \text{ Wanddicke}$$

6.

$$p(x,y) = \frac{H_{the\ best}(x,y) - H_m(x,y)}{\|H_{the\ best}(x,y) - H_m(x,y)\|}, \ \text{N} = 3 \text{ and } \Delta D = 5\% \text{ Wanddicke}$$

Die in Fig. 3a dargestellte Überwachungs-Routine 9 hat wie beschrieben insbesondere zwei Funktionen: Zum einen wird das Erreichen des Stopp-Kriteriums überprüft und zum zweiten werden die Ressourcen der EDV-Einheit zwischen den einzelnen Experten basierend auf deren Erfolgen vorgenommen. Ein Maß für den Erfolg ist

$$P = \frac{\Delta F}{N},$$

wobei ΔF die Reduktion der Fitnessfunktion F durch das Ergebnis der jeweiligen Experten-Routine und N die Anzahl der hierfür notwendigen Simulationen ist. Eine Bewertung der n Experten-Routinen kann als

$$R_n = \frac{P_n}{\sum P_i}.$$

angenommen werden. Die Anzahl der Rechen-Slots Ns für eine Experten-Routine in einer Iteration ist dann

$$\text{N}_\text{S} = \text{int}(R_\text{n}\ \text{N}_{all}),$$

wobei $N_{all}$ die Anzahl aller verfügbaren Slots ist.

In den Simulations-Routinen 16 wird eine MFL-Messung für eine Experten-Fehlstellengeometrie simuliert. Eine Experten-Routine kann solange iterieren, bis sie eine Lösung findet, deren Experten-Vorhersagedatensatz besser ist als der im Bereich 12 gespeicherte Ausgangsvorhersagedatensatz. Wenn dies der Fall ist, kann die Experten-Routine 11 einen weiteren linearen unabhängigen Datensatz bearbeiten oder ausgehend von der bereits verbesserten Lösung versuchen, weitere bessere Lösungen zu erreichen.

Sofern mehrere Datensätze aus verschiedenen Iterationen, die nicht übereinstimmend angeglichen werden können, von den Experten-Routinen durchgerechnet wurden, können ebenfalls automatisiert im Rahmen der Durchführung des erfindungsgemäßen Verfahrens die sich ergebenden Geometrien überlagert werden, wobei an den einzelnen Gitterpunkten als konservative Abschätzung die maximale Tiefe genommen wird:

$$D(x,y) = \max_N D_n(x,y)$$

für n = 1... N, wobei N die Anzahl der Datensätze ist, die nacheinander bearbeitet werden müssen. Ausgehend von einem auf einer solchen Überlagerung von Fehlstellengeometrien ergebenen resultierenden Tiefenprofil kann wiederum ein MFL-Signal simuliert werden. Der sich ergebende Fehler kann sich aus den Fehlern der jeweiligen Datensätzen in den einzelnen Kalkulationen ergeben:

$$E = \|H_{cal}(D) - H_m\|$$

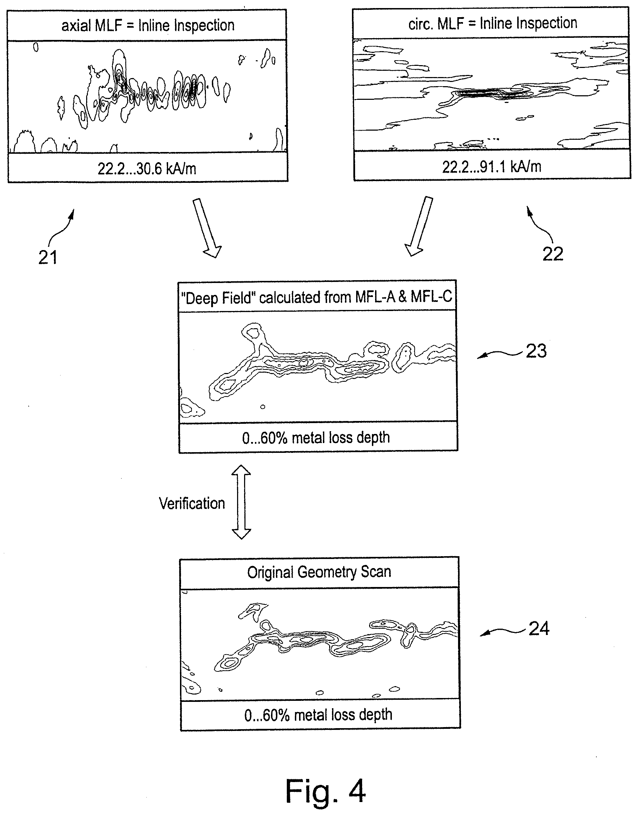

Um die Effizienz des vorgeschlagenen Verfahrens zu demontieren, wurde eine Vielzahl von Testszenarien durchgeführt, wobei nachfolgend gemäß Fig. 4 die Daten zweier MFL-Inspektionsläufe, die mit voneinander linear unabhängigen Magnetisieren durchgeführt wurden, verwendet werden.

Die Fig. 4 zeigt mit Nr. 21 beziffert eine Abbildung einer realen MFL-Messung mit in axialer Richtung verlaufender Magnetisierung, während die Abbildung 22 aus einer in Umfangsrichtung erfolgten Messung resultiert. Das Rechenergebnis der Fehlstellengeometrie, welches nach dem vorbeschriebenen, erfindungsgemäßen Verfahren erreicht wurde, ist mit 23 indiziert. Die Konturlinien teilen wie auch in Abbildung 24 den Bereich zwischen 0 und 60% Metallverlusttiefe gleichmäßig auf. Die Abbildung 24 zeigt die tatsächlich gescannte und damit direkt gemessene Außenoberfläche des zu den Abbildungen 21 und 22 zugehörigen Rohrabschnitts. Es ergibt sich eine sehr hohe Übereinstimmung der Laserscan-Messung mit der über das erfindungsgemäße Verfahren erreichten Lösung. Diese ist deutlich besser als die Lösung nach der im Stand der Technik bekannten Auswertung. Hierbei kann davon ausgegangen werden, dass die Abweichungen des Ergebnisses nach dem erfindungsgemäßen Verfahren und das der Laserscanmessung überwiegend aufgrund von technischen Toleranzen vorhanden sind.

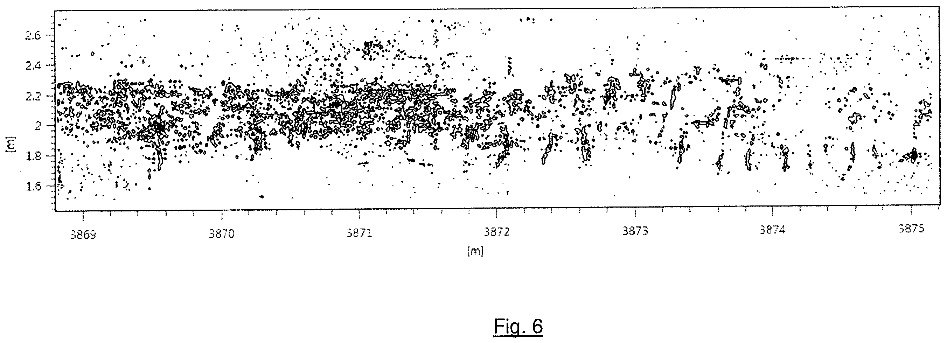

Auf Basis der herkömmlichen Betrachtung mit im Stand der Technik etablierter und in Fig. 1 im Ergebnis dargestellter Ermittlung der Fehlstellengeometrie ergibt sich der erwähnte maximum burst pressure von 4744,69 kPa. Auf Basis des erfindungsgemäßen Verfahrens ergibt sich für den auch der Fig. 1 zugrunde liegenden MFL-Datensatz die in der Fig. 6 gezeigte Fehlstellengeometrie (Konturlinien bei 2 mm Tiefe) und basierend hierauf ein maximum burst pressure von 8543,46 kPa. Dieser reicht vorliegend bis auf 99,4 % an den maximum burst pressure heran, der aufgrund der per Laserscan ermittelten tatsächlichen Fehlstellengeometrie bestimmt wurde. Demnach kann eine nach dem erfindungsgemäßen Verfahren untersuchte Pipeline mit einem sicheren Betriebsdruck von 6520,53 kPa betrieben werden. Hiermit ergeben sich im Vergleich zu dem sicheren Betriebsdruch von 3621,29 kPa aufgrund der Auswertung nach dem Stand der Technik (Fig. 1) erhebliche Vorteile für Pipeline-Betreiber. Aufgrund des erfindungsgemäßen Verfahrens lassen sich der Zustand eines Rohres und damit der für einen sicheren Betrieb der Pipeline angebbare Druck deutlich realistischer angeben, während die Betriebssicherheit nach wie vor gewährleistet ist. Durch das erfindungsgemäße Verfahren mit den um Ressourcen der EDV-Einheit konkurrierenden Experten-Routinen kann ein solches Ergebnis schneller oder zumindest in gleicher Auswertezeit wie im Stand der Technik den Betreibern von Pipelines zur Verfügung gestellt werden.

## Patentansprüche

1.  Verfahren zur Bestimmung der Geometrie einer oder mehrerer realer, untersuchter Fehlstellen eines magnetisierbaren Objekts, insbesondere eines Rohres oder eines Tanks, mit einem auf Basis einer oder mehrerer MFL-Messungen erzeugten Referenzdatensatz des Objekts,

    umfassend eine zumindest teilweise Darstellung des Objekts mittels einer EDV-Einheit, insbesondere auf einem oder durch ein zumindest dreidimensionales Objektgitter,
    sowie umfassend eine Bestimmung einer anfänglichen Fehlstellengeometrie als Ausgangsfehlstellengeometrie, insbesondere auf dem Objektgitter oder einem zumindest zweidimensionalen Defektgitter (15),
    eine Bestimmung eines ersten MFL-Vorhersagedatensatzes als Ausgangsvorhersagedatensatz auf Basis der Ausgangsfehlstellengeometrie, insbesondere durch Simulation einer MFL-Messung oder Zuweisung eines MFL-Datensatzes,
    und iterative Anpassung der Ausgangsfehlstellengeometrie an die Geometrie der realen Fehlstelle(n) mittels der EDV-Einheit und mittels mehrerer derart im Wettbewerb und vorzugsweise parallel zueinander laufender Experten-Routinen (11), dass die Experten-Routinen miteinander um Ressourcen der EDV-Einheit konkurrieren, wobei in jeweiligen Experten-Routinen (11) mittels wenigstens eines eigenen Algorithmus und auf Basis der Ausgangsfehlstellengeometrie eine jeweilige Experten-Fehlstellengeometrie erzeugt wird, auf Basis der jeweiligen Experten-Fehlstellengeometrie ein jeweiliger Experten-Vorhersagedatensatz, insbesondere durch Simulation einer MFL-Messung oder Zuweisung eines MFL-Datensatzes, bestimmt wird, und die dem jeweiligen Experten-Vorhersagedatensatz zugrunde liegende Experten-Fehlstellengeometrie dann wenigstens mehreren, insbesondere allen der Experten-Routinen (11) als neue Ausgangsfehlstellengeometrie zur weiteren Anpassung an die Geometrie der realen Fehlstelle(n) zur Verfügung gestellt wird, wenn der Experten-Vorhersagedatensatz dem Referenzdatensatz ähnlicher ist als der Ausgangsvorhersagedatensatz, und anschließend der zur neuen Ausgangsfehlstellengeometrie gehörende Experten-Vorhersagedatensatz als neuer Ausgangsvorhersagedatensatz verwendet wird, wobei die iterative Anpassung mittels der Experten-Routinen solange erfolgt, bis ein Stopp-Kriterium erfüllt ist.

2.  Verfahren nach Anspruch 1, **dadurch gekennzeichnet, dass** die Experten-Routinen (11) dergestalt im Wettbewerb zueinander laufen, dass eine Verteilung der Ressourcen der EDV-Einheit an eine jeweilige Experten-Routine insbesondere in Form von Rechenzeit, vorzugsweise CPU- und/oder GPU-Zeit, in Abhängigkeit einer Erfolgsquote, bei der insbesondere die Anzahl der von dieser Experten-Routine berechneten und für eine oder mehrere andere Experten-Routinen (11) zur Verfügung gestellten Ausgangsfehlstellengeometrien berücksichtigt wird, und/oder in Abhängigkeit einer Reduktion einer Fitnessfunktion, bei der insbesondere die Anzahl der für die Reduktion erzeugten Experten-Vorhersagedatensätze berücksichtigt wird, erfolgt.

3. Verfahren nach einem der vorherigen Ansprüche, **dadurch gekennzeichnet, dass** eine Fitnessfunktion als Maß für die Ähnlichkeit der Expertenvorhersage- und Referenzdatensätze verwendet wird.

4. Verfahren nach einem der vorherigen Ansprüche, **dadurch gekennzeichnet, dass** zur Bestimmung der Geometrie der Fehlstelle(n) zusätzlich ein von dem ersten Referenzdatensatz hinsichtlich der Magnetisierung linear unabhängiger weiterer Referenzdatensatz verwendet wird und auf Basis der Ausgangsfehlstellengeometrie ein weiterer Ausgangsvorhersagedatensatz insbesondere mittels einer die lineare Unabhängigkeit berücksichtigenden weiteren MFL-Simulation bestimmt wird und eine Experten-Fehlstellengeometrie erst dann als Ausgangsfehlstellengeometrie verwendet wird, wenn die für beide unabhängigen Magnetisierungen bestimmten zugehörigen Experten-Vorhersagedatensätze den jeweiligen Referenzdatensätzen ähnlicher sind als die für die beiden Magnetisierungen bestimmten Ausgangsvorhersagedatensätze und/oder eine beide Experten-Vorhersagedatensätze berücksichtigende Fitnessfunktion verbessert ist.

5. Verfahren nach Anspruch 4, **dadurch gekennzeichnet, dass** der erste Referenzdatensatz über eine MFL-Messung mit axialer Magnetisierung und der zweite Referenzdatensatz über eine MFL-Messung mit Magnetisierung in Umfangsrichtung des Rohres erzeugt wurde.

6. Verfahren nach einem der vorherigen Ansprüche, **dadurch gekennzeichnet, dass** Ausgangs- und/oder Experten-Vorhersagedatensätze auf Basis eines Vorwärtsmodels zur Simulation der MFL-Messung und insbesondere mittels eines Finite-Elemente-Models erzeugt werden.

7. Verfahren nach einem der vorherigen Ansprüche, **dadurch gekennzeichnet, dass** die anfängliche Fehlstellengeometrie mittels einer Look-up-Tabelle, durch eine der Experten-Routinen (11) und/oder durch einen maschinellen Lernalgorithmus erzeugt wird.

8. Verfahren nach einem der vorherigen Ansprüche, **dadurch gekennzeichnet, dass** das Defektgitter (5) in Bereichen, in denen die Tiefe der simulierten Fehlstelle(n) einen Schwellwert überschreitet, verfeinert wird.

9. Verfahren nach einem der vorherigen Ansprüche, **dadurch gekennzeichnet, dass** vor der Berechnung eines jeweiligen Experten-Vorhersagedatensatzes das Objekt- und/oder das Defektgitter (15) verfeinert wird.

10. Verfahren nach einem der vorherigen Unterschiede, **dadurch gekennzeichnet, dass** die Ausgangsfehlstellengeometrie oder ein hierauf verweisender Zeiger in einem für alle Experten-Routinen (11) zugänglichen Speicherbereich (12) der EDV-Einheit abgelegt wird.

11. Verfahren nach einem der vorherigen Ansprüche, **dadurch gekennzeichnet, dass** als Stopp-Kriterium eine nach einer Mehrzahl von Iterationen ausbleibende Änderung der Ausgangsfehlstellengeometrie und/oder der Geometrie des Objektund/oder Defektgitters (15) und/oder des Ausgangsvorhersagedatensatzes und/oder wenigstens eines Experten-Vorhersagedatensätze angenommen wird.

12. Verfahren nach Anspruch 11, **dadurch gekennzeichnet, dass** als Stopp-Kriterium ein Vergleich der Variation des Experten-Vorhersagedatensatzes mit der Messstreuung des realen Datensatzes verwendet wird.

13. Verfahren nach einem der vorherigen Ansprüche, **dadurch gekennzeichnet, dass** einer Experten-Routine (11) mehrere Algorithmen zur Anpassung der Experten-Fehlstellengeometrie umfassend maschinelles Lernen, stochastische Optimierung, empirische und/oder numerische Modellfunktionen zugeordnet sind.

14. Verfahren nach Anspruch 13, **dadurch gekennzeichnet, dass** in einer Experten-Routine (11) ein Algorithmus zufallsgeneriert oder durch eine Auswahlfunktion ausgewählt und/oder geändert wird.

15. Verfahren zur Bestimmung einer Belastbarkeitsgrenze eines zumindest im Betrieb druckbelasteten und insbesondere als Öl-, Gas- oder Wasserpipeline ausgebildeten Objekts, bei dem ein eine oder mehrere Fehlstelle(n) beschreibender Datensatz als Eingangsdatensatz in einer Berechnung der Belastbarkeitsgrenze verwendet wird, **dadurch gekennzeichnet, dass** der Eingangsdatensatz zunächst gemäß einem Verfahren nach einem der vorherigen Ansprüche bestimmt wird.

**EP 3 467 489 B1**

**Claims**

1. Method for determining the geometry of one or more real, examined defects of a magnetizable object, in particular a pipe or a tank, with a reference data set of the object generated on the basis of one or more MFL measurements,

   comprising an at least partial representation of the object via an EDP unit in particular on or via an at least three-dimensional object grid,
   and comprising a determination of an initial defect geometry as the initial defect geometry in particular on the object grid or an at least two-dimensional defect grid (15),
   a determination of a first MFL prediction data set as an initial prediction data set based on the initial defect geometry by simulating an MFL measurement or assigning an MFL data set,
   and iterative adaptation of the initial defect geometry to the geometry of the real defect(s) via the EDP unit and via several expert routines (11) running in competition and preferably in parallel with each other in such a way that the expert routines compete with each other for resources of the EDP unit,
   whereby a respective expert defect geometry is generated in respective expert routines (11) via at least one own algorithm and on the basis of the initial defect geometry,
   a respective expert prediction data set is determined on the basis of the respective expert defect geometry in particular by simulating an MFL measurement or assigning an MFL data set,
   and the expert defect geometry on which the respective expert prediction data set is based is then made available to at least several in particular all of the expert routines (11) as a new initial defect geometry for further adaptation to the geometry of the real defect(s),
   if the expert prediction data set is more similar to the reference data set than the initial prediction data set, and then the expert prediction data set belonging to the new initial defect geometry is used as the new initial prediction data set, whereby the iterative adjustment is carried out using the expert routines until a stop criterion is met.

2. Method according to claim 1, **characterized in that** the expert routines (11) run in competition with one another in such a way that the resources of the EDP unit are distributed to a respective expert routine in particular in the form of computing time, preferably CPU and/or GPU time, as a function of a success rate, in which in particular the number of initial defect geometries calculated by this expert routine and made available for one or more other expert routines (11) is taken into account, and/or as a function of a reduction of a fitness function, in which in particular the number of expert prediction data sets generated for the reduction is taken into account.

3. The method according to one of the preceding claims, **characterized in that** a fitness function is used as a measure of the similarity of the expert prediction and reference data sets.

4. Method according to one of the preceding claims, **characterized in that**, in order to determine the geometry of the defect(s), a further reference data set which is linearly independent of the first reference data set with respect to the magnetization is additionally used, and a further initial defect geometry is determined on the basis of the initial defect geometry in particular via a further MFL simulation, which takes into account the linear independence, and an expert defect geometry is only then used as the initial defect geometry, if the associated expert defect geometries determined for both independent magnetizations are more similar to the respective reference geometries than the initial defect geometries determined for the two magnetizations and/or a fitness function taking into account both expert defect geometries is improved.

5. Method according to claim 4, **characterized in that** the first reference data set was generated via an MFL measurement with axial magnetization and the second reference data set was generated via an MFL measurement with magnetization in the circumferential direction of the tube.

6. Method according to one of the preceding claims, **characterized in that** initial and/or expert prediction data sets are generated on the basis of a forward model for simulating the MFL measurement and in particular via a finite element model.

7. Method according to one of the preceding claims, **characterized in that** the initial defect geometry is generated via a look-up table, by one of the expert routines (11) and/or by a machine learning algorithm.

8. Method according to one of the preceding claims, **characterized in that** the defect grid (5) is refined in regions in which the depth of the simulated defect (s) exceeds a threshold value.

9. Method according to one of the preceding claims, **characterized in that** the object and/or defect grid (15) is refined before the calculation of a respective expert prediction data set.

10. Method according to one of the preceding differences, **characterized in that** the initial defect geometry or a pointer referring thereto is stored in a memory area (12) of the EDP unit accessible to all expert routines (11).

11. Method according to one of the preceding claims, **characterized in that** a change in the initial defect geometry and/or the geometry of the object and/or defect grid (15) and/or the initial prediction data set and/or at least one expert prediction data set which is absent after a plurality of iterations is assumed as a stop criterion.

12. Method according to claim 11, **characterized in that** a comparison of the variation of the expert prediction data set with the measurement scatter of the real data set is used as a stop criterion.

13. Method according to one of the preceding claims, **characterized in that** an expert routine (11) is assigned a plurality of algorithms for adapting the expert defect geometry comprising machine learning, stochastic optimization, empirical and/or numerical model functions.

14. Method according to claim 13, **characterized in that** an algorithm is randomly generated or selected and/or changed by a selection function in an expert routine (11).

15. Method for determining a load-bearing capacity limit of an object which is subjected to pressure at least during operation and is designed in particular as an oil, gas or water pipeline, in which a data set describing one or more defects is used as an input data set in a calculation of the load-bearing capacity limit, **characterized in that** the input data set is first determined in accordance with a method according to one of the preceding claims.

**Revendications**

1. Procédé permettant de déterminer la géométrie d'une ou de plusieurs imperfections examinées, réelles, d'un objet magnétisable, en particulier d'un tuyau ou d'un réservoir, avec un jeu de données de référence généré sur la base d'une ou de plusieurs mesures MFL de l'objet,

comprenant une représentation au moins partielle de l'objet au moyen d'une unité informatique, en particulier sur ou par une grille d'objet au moins tridimensionnelle, ainsi que comprenant une détermination d'une géométrie d'imperfection initiale en tant que géométrie d'imperfection de sortie, en particulier sur la grille d'objet ou une grille de défaut (15) au moins bidimensionnelle,
une détermination d'un premier jeu de données de prédiction MFL en tant que jeu de données de prédiction de sortie sur la base de la géométrie d'imperfection de sortie, en particulier par une simulation d'une mesure MFL ou l'attribution d'un jeu de données MFL,
et une adaptation itérative de la géométrie d'imperfection de sortie à la géométrie de la ou des imperfections réelles au moyen de l'unité informatique et au moyen de plusieurs routines expertes (11) s'exécutant en concurrence et de préférence en parallèle les unes aux autres de telle sorte que les routines expertes se font concurrence pour les ressources de l'unité informatique, dans lequel une géométrie d'imperfection experte est générée dans des routines expertes (11) respectives au moyen d'au moins un algorithme propre et sur la base de la géométrie d'imperfection de sortie,
un jeu de données de prédiction expert respectif est déterminé sur la base de la géométrie d'imperfection experte respective, en particulier par la simulation d'une mesure MFL ou l'attribution d'un jeu de données MFL,
et la géométrie d'imperfection experte à la base du jeu de données de prédiction expert respectif est mise à la disposition au moins de plusieurs, en particulier de toutes les routines expertes (11) en tant que nouvelle géométrie d'imperfection de sortie pour une adaptation supplémentaire à la géométrie de la ou des imperfections réelles,
lorsque le jeu de données de prédiction expert ressemble plus au jeu de données de référence que le jeu de données de prédiction de sortie, et ensuite le jeu de données de prédiction expert appartenant à la nouvelle géométrie d'imperfection de sortie est utilisé en tant que nouveau jeu de données de prédiction de sortie,
dans lequel l'adaptation itérative est effectuée au moyen des routines expertes jusqu'à ce qu'un critère d'arrêt soit satisfait.

2. Procédé selon la revendication 1, caractérisé en que les routines expertes (11) s'exécutent en concurrence les unes avec les autres de telle sorte qu'une distribution des ressources de l'unité informatique à une routine experte respective, en particulier sous la forme d'un temps de calcul, de préférence d'un temps de CPU et/ou de GPU, est effectuée en fonction d'un taux de réussite, dans laquelle en particulier le nombre de géométries d'imperfection de sortie calculées par cette routine experte et mises à la disposition d'une ou de plusieurs autres routines expertes (11) est pris en compte, et/ou est effectuée en fonction d'une réduction d'une fonction de pertinence dans laquelle en particulier le nombre des jeux de données de prédiction experts générés pour la réduction est pris en compte.

3.  Procédé selon l'une quelconque des revendications précédentes, **caractérisé en ce qu'**une fonction de pertinence est utilisée en tant que mesure de la similitude des jeux de données de prédiction experts et de référence.

4.  Procédé selon l'une quelconque des revendications précédentes, **caractérisé en ce que** pour la détermination de la géométrie de la ou des imperfections, en plus un jeu de données de référence supplémentaire, indépendant linéairement du premier jeu de données de référence en ce qui concerne la magnétisation, est utilisé, et sur la base de la géométrie d'imperfection de sortie, un jeu de données de prédiction de sortie supplémentaire est déterminé en particulier au moyen d'une simulation MFL supplémentaire tenant compte de l'indépendance linéaire, et une géométrie d'imperfection experte n'est utilisée en tant que géométrie d'imperfection de sortie que si les jeux de données de prédiction experts associés, déterminés pour les deux magnétisations indépendantes, ressemblent plus aux jeux de données de référence respectifs que les jeux de données de prédiction de sortie déterminés pour les deux magnétisations et/ou une fonction de pertinence tenant compte des deux jeux de données de prédiction experts est améliorée.

5.  Procédé selon la revendication 4, **caractérisé en ce que** le premier jeu de données de référence a été généré par l'intermédiaire d'une mesure MFL avec une magnétisation axiale, et le deuxième jeu de données de référence a été généré par l'intermédiaire d'une mesure MFL avec une magnétisation dans la direction circonférentielle du tuyau.

6.  Procédé selon l'une quelconque des revendications précédentes, **caractérisé en ce que** les jeux de données de prédiction de sortie et/ou experts sont générés sur la base d'un modèle direct pour la simulation de la mesure MFL et en particulier au moyen d'un modèle à éléments finis.

7.  Procédé selon l'une quelconque des revendications précédentes, **caractérisé en ce que** la géométrie d'imperfection initiale est générée au moyen d'une table de recherche par l'une des routines expertes (11) et/ou par un algorithme d'apprentissage machine.

8.  Procédé selon l'une quelconque des revendications précédentes, **caractérisé en ce que** la grille de défaut (5) est affinée dans des zones où la profondeur de la ou des imperfections simulées dépasse une valeur seuil.

9.  Procédé selon l'une quelconque des revendications précédentes, **caractérisé en ce que** la grille d'objet et/ou de défaut (15) est affinée avant le calcul d'un jeu de données de prédiction expert respectif.

10. Procédé selon l'une quelconque des différences précédentes, **caractérisé en ce que** la géométrie d'imperfection de sortie ou un pointeur s'y référant est sauvegardé(e) dans une zone de mémoire (12) accessible à toutes les routines expertes (11) de l'unité informatique.

11. Procédé selon l'une quelconque des revendications précédentes, **caractérisé en ce qu'**une absence de modification de la géométrie d'imperfection de sortie et/ou de la géométrie de la grille d'objet et/ou de défaut (15) et/ou du jeu de données de prédiction de sortie et/ou d'au moins un jeu de données de prédiction expert après une pluralité d'itérations est adoptée en tant que critère d'arrêt.

12. Procédé selon la revendication 11, **caractérisé en ce qu'**une comparaison de la variation du jeu de données de prédiction expert avec la dispersion de mesure du jeu de données réel est utilisée en tant que critère d'arrêt.

13. Procédé selon l'une quelconque des revendications précédentes, **caractérisé en ce que** plusieurs algorithmes d'adaptation de la géométrie d'imperfection experte, comprenant un apprentissage machine, une optimisation stochastique, des fonctions modèles empiriques et/ou numériques, sont attribués à une routine experte (11).

14. Procédé selon la revendication 13, **caractérisé en ce que** dans une routine experte (11), un algorithme est généré de manière aléatoire ou est sélectionné et/ou modifié par une fonction de sélection.

**15.** Procédé permettant de déterminer une limite de capacité de charge d'un objet sous pression au moins en cours de fonctionnement, et réalisé en particulier sous forme d'oléoduc, de gazoduc ou d'aqueduc, dans lequel un jeu de données décrivant une ou plusieurs imperfections est utilisé comme jeu de données d'entrée dans un calcul de la limite de capacité de charge, **caractérisé en ce que** le jeu de données d'entrée est d'abord déterminé selon un procédé selon l'une quelconque des revendications précédentes.

EP 3 467 489 B1

Fig. 1

18

Fig. 2

Fig. 3

| axial MLF = Inline Inspection |
|:---:|
| |
| 22.2...30.6 kA/m |

| circ. MLF = Inline Inspection |
|:---:|
| |
| 22.2...91.1 kA/m |

21

22

| "Deep Field" calculated from MFL-A & MFL-C |
|:---:|
| |
| 0...60% metal loss depth |

23

Verification

| Original Geometry Scan |
|:---:|
| |
| 0...60% metal loss depth |

24

Fig. 4

Fig. 5

Fig. 6

**EP 3 467 489 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20160187523 A1
- US 6316937 B1
- US 20160178580 A1
- US 20170176629 A1